# EUROPÄISCHE PATENTANMELDUNG

(11) **EP 3 753 619 A1**
(43) Veröffentlichungstag der Anmeldung: **23.12.2020**
(21) Anmeldenummer: 20180458.0
(22) Anmeldetag: 17.06.2020
(51) Int. Cl.: B01D 3/14, B01D 3/34, B01D 19/00, C07C 273/14, C07C 273/16

(54) **VERFAHREN ZUR KONTINUIERLICHEN HERSTELLUNG REINER HARNSTOFFLÖSUNGEN DURCH VERWERTUNG VON PROZESSWASSER**

(30) Priorität: 18.06.2019 DE 102019208859
(71) Anmelder: SKW STICKSTOFFWERKE PIESTERITZ GmbH, 06886 Lutherstadt Wittenberg (DE)
(72) Erfinder: Göhrmann, Bernd, 06749 Bitterfeld (DE); Ackermann, Andreas, 06889 Wittenberg (DE); Lippert, Roman, 06886 Wittenberg (DE); Geisler, Matthias, 06886 Wittenberg (DE)
(74) Vertreter: Grünecker Patent- und Rechtsanwälte PartG mbB

(57) **Zusammenfassung**

Die Erfindung betrifft ein Verfahren zur Herstellung reiner Harnstofflösungen und insbesondere einer reinen Harnstofflösung mit den Qualitätsmerkmalen von AUS 32 gemäß ISO 22241, in dem man eine nach einem herkömmlichen Harnstoffsyntheseverfahren erzeugte Harnstofflösung mit einem stöchiometrisch bei der Synthese entstehendem und nach einem Reinigungsverfahren mittels Desorption gereinigtem Prozesswasser in der Art vermischt, dass eine reine Harnstofflösung entsteht, die frei von harnstoffsynthese-typischen Verunreinigungen ist und weniger als 0,2 Gew.-% NH₃ und 0,3 Gew.-% Biuret, bezogen auf die Gesamtlösung, enthält.

## Beschreibung

Die Erfindung betrifft ein kontinuierliches Verfahren zur Herstellung reiner Harnstofflösungen und insbesondere einer Harnstofflösung (AUS 32 nach ISO 22241), die als Reduktionsmittel in einem auf selektiver katalytischer Reduktion basierenden Verfahren (selective catalytic reduction-SCR-Verfahren) eingesetzt wird.

Die Industrialisierung und das ständig steigende Verkehrsaufkommen erhöhen die Schadstoffkonzentration in der Umgebungsluft in bedenklichem Maße.

Einen großen Anteil haben Stickoxidemissionen, die auf die Verbrennung von Dieselkraftstoffen in Industrieanlagen und in Automobilen zurückzuführen sind.

Aus der DE-OS 3830045 ist bekannt, dass aus Harnstoff freigesetztes NH₃ als Reduktionsmittel für die selektive katalytische Reduktion von Stickoxiden in sauerstoffhaltigen Abgasen genutzt werden kann.

Entsprechend DE 10 2004 013 165 A1 wird dem Katalysator eine Harnstofflösung durch Eindüsen zugeführt, die häufig einen Harnstoffanteil von 32,5 % besitzt und unter der Bezeichnung AUS 32 bekannt ist.

Im SCR-Katalysator wird das im Abgas enthaltene NOₓ in die neutralen Komponenten (N₂, O₂, H₂O) umgesetzt.

In der DE 102 51 498 A1 wird darauf hingewiesen, dass die Herstellung von wässrigen Harnstofflösungen teuer ist, da sie zur Vermeidung von Ablagerungen am Katalysator mit deionisiertem Wasser hergestellt werden müssen, deshalb wird hier der Einsatz von festem Harnstoff beansprucht.

Darüber hinaus sind die Anforderungen an den aufzulösenden Harnstoff groß. Üblicher Düngeharnstoff (Granulat, Prills) kann häufig nicht verwendet werden, da er mit dem Anticaking-Mittel Formalin behandelt ist und für die Harnstoffherstellung typische Verunreinigungen wie Schwermetalle und Öl enthält.

Schwermetalle und Öle verkürzen die Laufzeit der Katalysatoren.

DE 10 2006 059 760 B4 trifft die Aussage, dass Harnstofflösungen, die direkt in Harnstoffprozessanlagen, wie in DE-AS 2015781, DE-AS 1913121, DE-AS 2411205 beschrieben, in den Prozessstufen Synthese und Rezirkulation anfallen, den hohen Qualitätsmaßstäben nicht genügen.

DE 10 2006 059 760 B4 beschreibt ein energieaufwändiges Verfahren, bei dem Harnstofflösung kristallisiert, getrocknet und wieder zur entsprechenden Lösung mit Dampfkondensaten vermischt wird.

EP 0 053 410 bescheibt ein Verfahren zum Entfernen von Harnstoff, Ammoniak und Kohlendioxid aus verdünnten wässrigen Lösungen.

US 10,280,094 betrifft ein Verfahren zur Behandlung von wässrigen Lösungen, die geringe Mengen an Harnstoff, Ammoniak und Kohlendioxid enthalten.

DE 25 59 112 bescheibt ein Verfahren zur Behandlung von Wasserdampf, der geringe Mengen an Ammoniak und Kohlendioxid enthält und der beim Konzentrieren einer wässrigen Harnstofflösung erzeugt worden ist.

Eine nach einem herkömmlichen Harnstoffsyntheseverfahren erzeugte Harnstofflösung enthält 72±5 Gew.-% Harnstoff, 27±5 Gew.-% Wasser, 0,3-0,6 Gew.-% Biuret, 0,1-0,2 Gew.-% NH₃ und 0,1-0,2 Gew-% CO₂, bezogen auf die Gesamtlösung.

Der Erfindung liegt die Aufgabe zugrunde, ein Verfahren zu schaffen, mit dem energetisch hocheffektiv eine reine Harnstofflösung kontinuierlich produziert werden kann, die als Reduktionsmittel für die selektive katalytische Reduktion von Stickoxiden geeignet ist.

Diese Aufgabe wird erfindungsgemäß mit einem Verfahren mit den Merkmalen des Anspruches 6 gelöst, in dem man eine nach einem herkömmlichen Harnstoffsyntheseverfahren erzeugte Harnstofflösung mit einem stöchiometrisch bei der Synthese entstehendem und nach einem Verfahren gemäß der Ansprüche 1 bis 5 gereinigtem Prozesswasser in der Art vermischt, dass eine reine Harnstofflösung entsteht, die frei von harnstoffsynthese-typischen Verunreinigungen ist und weniger als 0,2 Gew.-% NH₃ und 0,3 Gew.-% Biuret, bezogen auf die Gesamtlösung, enthält.

Die Herstellung reiner Harnstofflösungen nach Anspruch 6 schließt erfindungsgemäß die Herstellung einer reinen Harnstofflösung ein, die die Norm der ISO 22241 erfüllt.

Überraschenderweise wurde gefunden, dass in der üblichen Harnstoffsynthese anfallendes Prozesswasser mit dem Verfahren nach Anspruch 1 bis 5 aufgereinigt werden kann und verwendet werden kann, um entsprechend Anspruch 9 eine Harnstofflösung herzustellen, die die Anforderungen der ISO 22241 erfüllt.

Die Synthese von Harnstoff ist dem Fachmann bekannt. Die industrielle Herstellung erfolgt aus NH₃ und CO₂, wobei in einem ersten Schritt Ammoniumcarbamat gebildet wird, was weiter zu Harnstoff und Wasser reagiert.

2NHₐ + CO₂ ⇄ H₂NCOONH₄ ⇄ (NH₂)₂CO + H₂O

Entsprechend der Reaktionsgleichung entsteht bei der Synthese aus den Grundstoffen pro Mol Harnstoff ein Mol Wasser. Dieses Wasser ist bei Anfall durch prozessbedingte Verunreinigungen nicht für die Herstellung von reinen Harnstofflösungen geeignet. Ziel der Erfindung ist es, dieses Wasser für die Herstellung von reinen Harnstofflösungen dennoch zu verwenden. Entsprechend wird dieses Wasser soweit gereinigt, dass es auch für die Herstellung einer reinen Harnstofflösung entsprechend ISO 22241 geeignet ist.

Das entsprechend der Reaktionsgleichung entstehende Wasser wird zum Teil abgetrennt, so dass eine Harnstofflösung erhalten wird, die 72±5 Gew.-% Harnstoff und 27±5 Gew.-% Wasser sowie weitere während der Synthese anfallende Nebenprodukte enthält. Das abgetrennte Wasser wird im Folgenden Prozesswasser genannt.

Gemäß dem erfindungsgemäßen Verfahren wird das in der herkömmlichen Harnstoffsynthese anfallende Prozesswasser durch folgende Schritte aufgereingt. In einer ersten Reinigungskolonne wird das Prozesswasser bei Temperaturen von 100 bis 150 °C durch Einleiten von Dampf oder vorzugsweise von Entspannungsgasen, bestehend aus Ammoniak, Kohlendioxid und Wasserdampf aus den Reinigungskolonnen 2 und 3 auf 100 bis 150 °C aufgeheizt und anschließend kondensiert. In diesem ersten Reinigungsschritt wird das Prozesswasser von im Wasser gelösten NH₃ und CO₂ gereinigt.

In einer bevorzugten Ausführungsform in Verbindung mit einer der oben oder unten genannten Ausführungsformen wird das Gemisch aus Prozesswasser und Entspannungsgas auf 130 bis 150 °C aufgeheizt, besonders bevorzugt 140 bis 150 °C, insbesondere 150 °C.

Das so erhaltene und von gelöstem NH₃ und CO₂ befreite Prozesswasser wird anschließend einem zweiten Reinigungsschritt in einer zweiten Reinigungskolonne unterworfen, wobei mittels Wasserdampf unter einem Druck von 5 bis 15 bar restliche im Wasser vorhandene Harnstoffverbindungen in NH₃ und CO₂ gespalten werden. Die Gase (NH₃ und CO₂) werden durch Entspannung ausgetrieben und in die erste Reinigungsstufe zurückgeführt.

In einer bevorzugten Ausführungsform in Verbindung mit einer der oben oder unten genannten Ausführungsformen wird der zweite Reinigungsschritt bei einer Temperatur von 100 bis 150 °C, besonders bevorzugt 130 bis 150 °C, insbesondere 140 bis 150 °C durchgeführt. Der Druck im zweiten Reinigungsschritt beträgt in einer bevorzugten Ausführungsform in Verbindung mit einer der oben oder unten genannten Ausführungsform 7 bis 14 bar, besonders bevorzugt 8 bis 12 bar, insbesondere 9 bis 11 bar.

Das so erhaltene gereinigte Prozesswasser wird anschließend einem dritten Reinigungsschritt unterworfen, wobei das Kondensat aus der zweiten Reinigungskolonne in eine dritte Reinigungskolonne überführt wird und mit Wasserdampf im Gegenstrom von Resten von NH₃ und CO₂ befreit wird.

In einer bevorzugten Ausführungsform in Verbindung mit einer der oben oder unten genannten Ausführungsformen wird der dritte Reinigungsschritt bei einer Temperatur von 100 bis 150 °C, besonders bevorzugt 130 bis 150 °C, insbesondere 140 bis 150 °C durchgeführt.

Das so gereinigte Prozesswasser weist eine Leitfähigkeit von ≤ 10 µS/cm auf und kann somit verwendet werden, um reine Harnstofflösungen mit den gewünschten Konzentrationen herzustellen, insbesondere Harnstofflösungen, die die Reinheitsanforderungen der ISO 22241 erfüllen.

Entsprechend dem erfindungsgemäßen Verfahren wird die aus dem Harnstoffprozess erhaltene Harnstofflösung mit 72±5 Gew.-% Harnstoff und 27±5 Gew.-% Wasser einer Mischstrecke zugeführt. Das nach dem oben beschriebenen Verfahren aufgereinigte Prozesswasser wird ebenfalls dieser Mischstrecke zugeführt.

In der Mischstrecke wird über temperaturkompensierte Durchflussmesser eine Harnstofflösung mit 30bis 50 Gew.-% Harnstoff, bezogen auf die Gesamtlösung, vorzugsweise 32 bis 38 Gew.-Harnstoff, bezogen auf die Gesamtlösung, eingestellt. Diese kann anschließend über einen Wärmetauscher gekühlt werden.

In einer weiteren bevorzugten Ausführungsform in Verbindung mit einer der oben oder unten genannten Ausführungsformen wird im Folgenden die Harnstofflösung nach dem Vermischen mit dem gereinigten Prozesswasser über einen geeigneten Koaleszenzabscheider von Öl und Schmutz weiter gereinigt

Die gereinigte Harnstofflösung gelangt in einen Rührbehälter. Hier erfolgt die Feineinstellung des Gehaltes durch Zugabe von weiterem Prozesswasser und/oder Dampfkondensat. Zur Bestimmung der Konzentration eignen sich erfindungsgemäß kontinuierliche physikalische Analysenmessungen, wie Dichte-, bzw. Brechzahlmessung, die unter Beachtung der Temperaturabhängigkeit als Konzentrationsmessung kalibriert werden müssen.

Entsprechend dem erfindungsgemäßen Anspruch 8 wird die Harnstofflösung mit dem gereinigten Prozesswasser vermischt auf eine Konzentration von 31,8 bis 33,2 Gew.-%, bezogen auf die Gesamtlösung.

In herkömmlichen Harnstoffsyntheseverfahren erzeugte Harnstofflösungen werden üblicherweise in einer Syntheseanlage mit Duplexstählen (z.B. Safurex®) als Material für die Hauptausrüstung im Syntheseteil und hocheffektiven Druckerhöhungspumpen für Ausgangsstoffe und Zwischenprodukte, welche den Eintrag von Fremdstoffen weitestgehend ausschließen, hergestellt.

Übliche harnstoffsynthese-typische Verunreinigungen sind NH₃, CO₂ und Biuret.

Die nach dem erfindungsgemäßen Verfahren erhaltene reine Harnstofflösung ist weitestgehend von diesen Verunreinigungen befreit und enthält weniger als 0,2 Gew.-% NH₃ und 0,3 Gew.-% Biuret, bezogen auf die Gesamtlösung.

Die insbesondere entsprechend Anspruch 9 hergestellte reine Harnstofflösungerfüllt die Qualitätskriterien von AUS 32 gemäß ISO 22241 und entspricht folgenden Qualitätsparametern:

| | |
|---|---|
| Ammoniak | < 0,2 Gew.-% bezogen auf die reine Harnstofflösung |
| Biuret | < 0,3 Gew.-% bezogen auf die reine Harnstofflösung |
| ungelöste Bestandteile | < 20 mg/kg bezogen auf die reine Harnstofflösung |

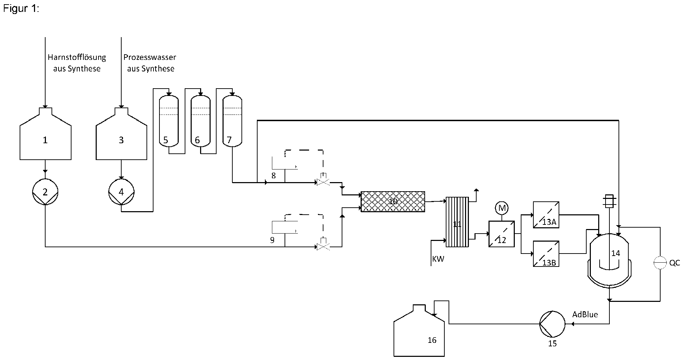

Die Erfindung wird durch die Zeichnung (Figur 1) näher veranschaulicht, die eine bevorzugte Ausführungsform an Hand eines Flussdiagrammes beispielhaft wiedergibt.

Zwischen 10-35 t/h Harnstofflösung aus Tank 1 werden mit 12-45 t/h Prozesswasser aus Tank 3, das in den Kolonnen 5, 6, 7 entsprechend Anspruch 1 bis 5 aufgereinigt wurde, im statischen Mischer 10 vermischt.

Die Dosierung erfolgt dabei über die Mengenregler 8 und 9, die entstandene Lösung wird im Wärmetauscher 11 mit Kühlwasser gekühlt und über die Filter 12 und 13 A/B gefiltert. Filter 12 fungiert dabei noch als Koaleszenzabscheider zur Ölabtrennung.

Im Rührapparat 14 wird über eine Qualitätsmessung die gewünschte Harnstoffkonzentration, z.B. von 32,5 Gew.-%, bezogen auf die Gesamtlösung, genau eingeregelt. Die Feineinstellung erfolgt über Zugabe von Prozesswasser und/oder Dampfkondensat in 14. Über die Pumpe 15 erfolgt die Ausspeicherung in die Großtanks 16.

Das erfindungsgemäße Verfahren stellt ein effizientes Verfahren zum Herstellen reiner Harnstofflösungen bereit, wobei das in der Harnstoffsynthese anfallende Prozesswasser verwendet werden kann, um eine Harnstofflösung mit den gewünschten Harnstoffkonzentrationen zu erhalten.

## Patentansprüche

1. Verfahren zum Aufreinigen von Prozesswasser aus der Harnstoffsynthese, **dadurch gekennzeichnet, dass** das Verfahren folgenden Schritte umfasst:
a. Einleiten heißer Entspannungsgase oder reinen Wasserdampfes und Aufheizen auf 100 bis 150 °C in einer ersten Reinigungskolonne;
b. Einleiten von Wasserdampf bei einem Druck von 5 bis 15 bar in einer zweiten Reinigungskolonne; und
c. Reinigung mit Wasserdampf in einer dritten Reinigungskolonne im Gegenstrom.

2. Verfahren nach Anspruch 1, **dadurch gekennzeichnet, dass** die Temperatur in der ersten Reinigungskolonne 130 bis 150 °C beträgt.

3. Verfahren nach Anspruch 1 oder 2, **dadurch gekennzeichnet, dass** die Temperatur in der zweiten Reinigungskolonne 130 bis 150 °C beträgt

4. Verfahren nach einem der Ansprüche 1 bis 3, **dadurch gekennzeichnet, dass** der Druck in der zweiten Reinigungskolonne 8 bis 12 bar beträgt.

5. Verfahren nach einem der Ansprüche 1 bis 4, **dadurch gekennzeichnet, dass** die Temperatur in der dritten Reinigungskolonne 100 bis 150 °C beträgt.

6. Verfahren zur kontinuierlichen Herstellung reiner Harnstofflösungen aus nach einem herkömmlichen Harnstoffsyntheseverfahren erzeugten Harnstofflösungen, **dadurch gekennzeichnet, dass** Harnstofflösung direkt aus dem Harnstofflösungstank gespeist und mit stöchiometrisch bei der Synthese entstehendem und nach dem Verfahren nach einem der Ansprüche 1 bis 5 gereinigtem Prozesswasser in der Art vermischt wird, dass eine reine Harnstofflösung entsteht, die frei von harnstoffsynthese-typischen Verunreinigungen ist und weniger als 0,2 Gew.-% NH₃ und 0,3 Gew.-% Biuret enthält, bezogen auf die Gesamtlösung.

7. Verfahren nach Anspruch 6, **dadurch gekennzeichnet, dass** die Harnstofflösung nach dem Vermischen mit gereinigtem Prozesswasser über einen geeigneten Koaleszenzabscheider von Öl und Schmutz weiter gereinigt wird.

8. Verfahren nach einem der Ansprüche 1 bis 7, **dadurch gekennzeichnet, dass** die Harnstofflösung mit dem gereinigten Prozesswasser auf eine Konzentration von 31,8 bis 33,2 Gew.-%, bezogen auf die Gesamtlösung, vermischt wird.

9. Verfahren nach einem der Ansprüche 1 bis 8, **dadurch gekennzeichnet, dass** die Harnstofflösung die Norm der ISO 22241 erfüllt.
